Europäisches Patentamt

European Patent Office

Office européen des brevets

(11) Veröffentlichungsnummer: **0 220 591 B1**

(12) **EUROPÄISCHE PATENTSCHRIFT**

(45) Veröffentlichungstag der Patentschrift: **27.12.90**

(21) Anmeldenummer: **86114162.0**

(22) Anmeldetag: **13.10.86**

(51) Int. Cl.⁵: **C 12 M 1/04, B 01 D 19/00**

(54) **Fermentationsanlage.**

(30) Priorität: **28.10.85 CH 4632/85**

(43) Veröffentlichungstag der Anmeldung:
**06.05.87 Patentblatt 87/19**

(45) Bekanntmachung des Hinweises auf die
Patenterteilung:
**27.12.90 Patentblatt 90/52**

(84) Benannte Vertragsstaaten:
**AT BE CH DE ES FR GB IT LI NL SE**

(56) Entgegenhaltungen:
EP-A-0 015 680     FR-A-1 584 983
DE-C- 308 288     FR-A-2 118 609
FR-A-1 516 392     US-A-3 488 926

PATENTS ABSTRACTS OF JAPAN, Band 5, Nr.
144 (C-71)816r, 11. September 1981; & JP-A-56
76 213 (MITSUBISHI JUKOGYO K.K.) 23-06-1981

**Die Akte enthält technische Angaben, die nach
dem Eingang der Anmeldung eingereicht
wurden und die nicht in dieser Patentschrift
enthalten sind.**

(73) Patentinhaber: **GEBRÜDER SULZER
AKTIENGESELLSCHAFT
Zürcherstrasse 9
CH-8401 Winterthur (CH)**

(72) Erfinder: **Ziegler, Heinrich, Dr.
Im Baumgarten
CH-8479 Rutschwil (CH)**

(74) Vertreter: **Sparing Röhl Henseler Patentanwälte
European Patent Attorneys
Rethelstrasse 123
D-4000 Düsseldorf 1 (DE)**

Courier Press, Leamington Spa, England.

**Beschreibung**

Die Erfindung betrifft eine Fermentationsanlage, aufweisend eine Rohrschlaufe für einen durch eine Umwälzpumpe erzwungenen Kreislauf eines flüssigen, hochviskosen, nicht-newton'schen (pseudoplastischen) Fermentationsgemisches, welche einen Abschnitt zum Begasen und Mischen des Fermentationsgemisches, einen Abschnitt zum Abscheiden eines bei dem Begasen und Fermentieren entstehenden Gasgemisches aus dem Fermentationsgemisch und eine Vorrichtung zum Abführen des Gasgemisches aus der Fermentationsanlage aufweist. Der Begriff "pseudoplastische Flüssigkeiten bzw. Gemische" soll alle Flüssigkeiten einschliessen, bei denen Scherkräfte eine "Verflüssigung", d.h. eine Erniedrigung ihrer Viskosität bewirken, unabhängig davon, ob diese Wirkung unmittelbar oder erst nach Ueberschreiten eines Schwellenwertes für die Scherkraft eintritt und unabhängig davon, ob bei Nachlassen der Scherkraft eine Hysterese auftritt oder nicht.

Solche Fermentationsanlagen sind zur Durchführung aerober Fermentationsprozesse bekannt. In dem begasten Rohrabschnitt wird durch das Begasen und Mischen der notwendige Gas/Flüssig-Sauerstoffübergang und die entsprechende Kohlendioxyd-Desorption durchgeführt. In einer Gas/Flüssig-Trenneinheit wird die an Sauerstoff verarmte und mit Kohlendioxyd angereicherte Gasphase aus dem Fermentationsgemisch weitgehend abgetrennt. Für hochviskose, nicht-newton'sche Fermentationsgemische, wie sie häufig z.B. bei der Herstellung von Antibiotika und Enzymen, sowie bei der Produktion von biologischen Polymeren, z.B. von Xanthan auftreten, ist es einem Fachmann in diesem Gebiet bekannt und verfügbar, den begasten Rohrabschnitt mit Einbauten, beispielsweise statischen Mischelementen, auszurüsten, um die eingetragene Energie über den ganzen Rohrquerschnitt homogen zu dissipieren und so eine homogene Dispersion der Gasphase und einen intensiven Stoffaustausch in dem Fermentationsgemisch zu erzielen.

Im Falle der hochviskosen, nicht-newton'schen (pseudoplastischen) Fermentationsgemische besteht ein hauptsächliches Problem in der erwähnten Abtrennung der Gasphase aus dem Fermentationsgemisch. Dies muss unter sterilen Bedingungen geschehen und mit einer kurzen Verweilzeit in der Trennvorrichtung. Gebräuchliche Gas/Flüssig-Trenneinheiten, wie sie in der Fachliteratur beschrieben oder empfohlen werden, vermögen die gestellte Aufgabe nicht zu lösen.

Anordnungen, die auf der Trennwirkung aufgrund der Verlangsamung der Flüssigkeitsgeschwindigkeit beruhen, sind ungeeignet, da der Auftrieb der Gasblasen zu gering ist, um durch eine Schicht der hochviskosen Flüssigkeit bzw. Suspension aufzusteigen. Darüberhinaus bewirkt die Verlangsamung in derartigen Anordnungen bei pseudoplastischen Flüssigkeiten eine Erhöhung der scheinbaren Viskosität der flüssigen Phase, wodurch eine Entgasung eher erschwert wird.

Andererseits sind Trennvorrichtungen, die auf Einwirkung der Zentrifugelkraft beruhen (Zyklone und ähnliche) nicht in der Lage das vorliegende Trennproblem zu lösen, weil die Geschwindigkeit der Flüssigkeit nach dem Eintritt in die Einheit infolge der erhöhten Viskosität schnell abnimmt und so die erwartete Funktionsweise einer solchen Trennvorrichtung nie zum Tragen kommt.

Weiterhin sind aus der EP-A 0 015 680 Gas/Flüssigkeits-Trennvorrichtungen für niedrigviskose newton'sche Flüssigkeiten bekannt; durch Einbauten soll bei diesen bekannten Vorrichtungen die Koaleszenz der Gasblasen gefördert werden. Durch diese Einbauten werden jedoch praktisch keine Scherkräfte auf die Flüssigkeit übertragen, so dass dadurch nicht-newton'sche Flüssigkeiten in ihrer Viskosität nicht beeinflusst werden.

Die FR-A-1 516 392 betrifft eine Trennvorrichtung für Erdoel, also ebenfalls eine newton'sche Flüssigkeit; in dieser Vorrichtung sind Einbauten mit engen Kanälen vorgesehen, die nach oben abgedeckt sind. Auch diese Einbauten dienen zur Förderung der Koaleszenz der Gasblasen. Zwar werden in den engen Kanälen bei nicht-newton'schen Flüssigkeiten Scherkräfte erzeugt und damit Viskositätserniedrigungen bewirkt. Die dadurch freigesetzten Gase können jedoch nicht aus der Flüssigkeit entweichen, so dass sie sehr weitgehend wieder redispergiert werden. Nicht-newton'sche Flüssigkeiten können in dieser Vorrichtung somit nicht wirksam entgast werden.

Der Erfindung liegt die Aufgabe zugrunde, für die Fermentationsanlage der anfangs beschriebenen Art eine Gas/Flüssigkeits-Trennvorrichtung zu entwerfen, die im falle der Behandlung von hochviskosen, nicht-newton'schen Gas/Flüssig-Gemischen verlässlich die Gasphase aus dem Gemisch entfernt, und das während kurzer Verweilzeit unter sterilen Bedingungen und mit einfachen, keine grosse Ueberwachung benötigenden Mitteln.

Diese Aufgabe ist erfindungsgemäss dadurch erfüllt, dass in dem Abschnitt zum Abscheiden des Gasgemisches Einbauten vorgesehen sind zum Scheren des Gas/Flüssigkeit-Gemisches in engen, vertikalen, nach oben offenen Gängen zum Herabsetzen der Viskosität des Gemisches, so dass die im Gemisch dispergierten Gase durch die Gemisch-Schicht mit eigenem Auftrieb im Raum nach oben steigen in einen Sammelraum, aus welchem sie durch die Vorrichtung zum Abführen des Gasgemisches aus dem Kreislauf weggeleitet werden.

Die erfindungsgemässe Lösung des Abscheidens der Gasphase beruht auf der Tatsache, dass die Viskosität solcher hochviskosen, nicht-newton'schen Fermentationsgemische durch intensive Einwirkung von Scherkräften beim Durchlauf des Gemisches durch die engen, nach oben geöffneten Gänge herabgesetzt wird, so dass bereits der natürliche Auftrieb von Gasblasen auch in diesen hochviskosen Fermentationsgemischen genügt, um ein Aufsteigen der Gasblasen und Bilden grösserer Gasagglomerationen zu erreichen.

In den Unteransprüchen werden bevorzugte Ausführungsformen der Erfindung angegeben.

Im weiteren wird die Erfindung und mit ihr erzielbare Vorteile näher beschrieben und erklärt. Die Beschreibung bezieht sich auf eine Zeichnung, in welcher zeigen:

Figur 1 eine erfindungsgemässe Fermentationsanlage in schematischer Darstellung,

Figur 2 eine erste Baueinheit im axialen Schnitt aus der Perspektive des Pfeiles A in Figur 1 in vergrössertem Massstab,

Figur 3 einen Querschnitt durch die erste Baueinheit gemäss Linie B/B in Figur 2,

Figur 4 eine zweite Baueinheit teilweise im Schnitt und im vergrösserten Massstab.

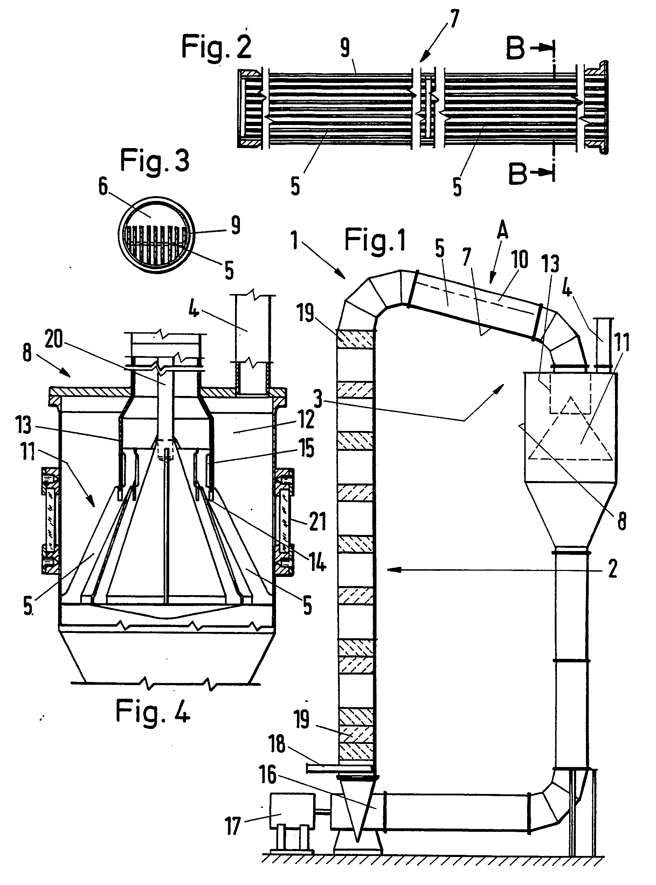

Die zur Fermentation von hochviskosen, nicht-newton'schen (pseudoplastischen) Fermentationsgemischen entworfene Anlage ist im wesentlichen eine Rohrschlaufe 1, in welcher die Fermentation beim Umwälzen des Gemisches in einem Kreislauf vor sich geht. Die Umwälzung im Kreislauf ist mittels einer Umwälzpumpe 16 erzwungen, die mit einem Motor 17 angetrieben ist. Der zu der Fermentation notwendige Sauerstoff wird in die Rohrschlaufe mit Einblasen von Luft durch ein Rohr 18 eingeblasen. Die Einblasestelle befindet sich am Anfang des Abschnitts 2 der Rohrschlaufe 1. In diesem Abschnitt kommt es zu der Vermischung des Fermentationsstoffes mit der durch die Leitung 18 eingeführten, steril filtrierten Luft. Durch den Einsatz von statischen Mischelementen 19, die in dem Schema schraffiert in dem Rohrabschnitt 2 angedeutet sind, wird die eingetragene Energie über den ganzen Rohrquerschnitt dissipiert und dadurch wird eine homogene Dispersion der Gasphase und ein intensiver Stoffaustausch in dem zu behandelnden, fermentierenden Gemisch erreicht. Bei der beschriebenen Reaktion entstehen Gase, die aus dem Fermentationsgemisch ständig, d.h. bei jeder Umwälzung abgeschieden werden müssen. Dies geschieht in der Fermentationsanlage in dem Abschnitt 3, der zum Abscheiden des bei dem Begasen und Fermentieren entstehenden Gasgemisches aus dem Fermentationsgemisch eingerichtet ist. Das hier aus dem Gemisch abgeschiedene Gasgemisch wird aus diesem Abschnitt 3 durch eine Vorrichtung 4 zum Abführen des Gasgemisches aus der Fermentationsanlage weggeleitet. Das in dieser Abscheidevorrichtung 3 entgaste Fermentationsgemisch wird in den Kreislauf zurück zu der Pumpe 16 geführt und wieder in den Abschnitt 2 zum Begasen und Mischen geschickt. Bei der Darstellung der erfindungsgemässen Fermentationsanlage in Figur 1 wurden sämtliche sonst noch bei einer solchen Anlage üblichen Einrichtungen wegen Vereinfachung ausgelassen. Es würde sich vornehmlich handeln um Abgaskühler, Sterilfilter für Luft, verschiedene Wärmetauscher zum Beheizen bzw. Kühlen oder Temperaturhalten des Fermentationsgemisches, verschiedene Dosiereinrichtungen zum Zuführen von Additivstoffen, wie Säure, Lauge oder Antischaumstoffen zu der Fermentation sowie verschiedene Messund Reguliereinrichtungen. Es ist auch nicht gezeigt, dass die Vorrichtung 4 zum Abführen des Gasgemisches aus dem Kreislauf mit einem Sterilfilter versehen ist und gegebenenfalls zur Abgasanalyse führt.

Das Abscheiden von Gasen geschieht in dem Abschnitt 3, in dem Einbauten 5 vorgesehen sind zum Scheren des Gas/Flüssig-Gemisches in engen, vertikalen, nach oben offenen Gängen. In diesen engen, nach oben offenen Gängen kommt es zum Herabsetzen der Viskosität des Gemisches durch die scherende Reibung an der Vielzahl von Wänden zwischen denen das Gemisch gezwungenerweise, angetrieben durch die Einwirkung der Umwälzpumpe passieren muss. Dabei kommt es durch das Scheren zum Herabsetzen der Viskosität des Gemisches zu solchem Mass, so dass die im Gemisch dispergierten Gase in der Lage sind, durch die Gemischschicht mit eigenem Auftrieb im Raum nach oben zu steigen in einen Sammelraum 6, 10, 12. In diesem Sammelraum wird das Gemisch praktisch keinen Scherkräften mehr ausgesetzt, so dass die in diesen Sammelraum aufgestiegenen Blasen anwachsen, grössere Gasagglomerationen bilden und sich schliesslich von der Flüssigkeit ablösen. Wie es besonders in den Figuren 2, 3 und 4 dargestellt ist, sind die oben erwähnten Einbauten im wesentlichen vertikal im Raum angeordnete Wände 5, zwischen denen die engen, nach oben offenen Gänge gebildet sind. Bei einer bereits ausgeführten Versuchsanlage wurden die Wände 5 zueinander in einem Abstand von 10 mm angeordnet. Ganz allgemein wird der Abstand zwischen den einzelnen Wänden vorteilhafterweise prozess-spezifisch gewählt, d.h. vor allem in Abhängigkeit vom Viskositätsverhalten des zu entgasenden Gemisches und vom gewünschten Entgasungsgrad. Entsprechend dem physikalischen Verhalten pseudoplastischer Gemische wird eine ins Gewicht fallende Erniedrigung der scheinbaren Viskosität nur innerhalb der wandnahen Grenzschicht erzielt, womit auch eine wirksame Entgasung nur in diesem Bereich möglich ist. Die Grenzschicht-Dicke ist vom rheologischen (Fliess-) Verhalten der jeweiligen Flüssigkeit oder des jeweiligen Gemisches abhängig. Eine optimale Entgasung wird erzielt, wenn die Kanalweite angenähert der doppelten Grenzschicht-Dicke entspricht. Diese zu verwirklichen kann in der Praxis jedoch, abhängig vom rheologischen Verhalten des Fermentationsgemisches, nur schwer realisierbar, unwirtschaftlich oder unnötig sein. In der bereits erwähnten Anlage, die zur Produktion eine Biopolymeres eingesetzt wurde, gelang es beim erwähnten Abstand der Wände 5 den Gasvolumen Anteil in Gemisch konstant auf kleiner 2% zu senken. Die flüssige Phase enthielt dabei eine Konzentration von 40 g/l an Biopolymeren, was zu typisch pseudoplastischem Fliessverhalten führte. Dabei lagen die in einem Zylinder-Viskosimeter (Contraves Rheomat 15) gemessenen scheinbaren Viskositäten bei einem Schergefälle D von 700 s$^{-1}$ bei

0,12 Pa s, bei einem D von 200 s⁻¹ bei 0,55 Pa s und bei einem D von 27 s⁻¹ bei 8,25 Pa s.

Wie es besonders gut in der Figur 3 zu sehen ist, reichen die vertikalen Wände 5 nicht bis in den oberen Teil des betreffenden Raumes, sondern es wird oberhalb der oberen Kanten der Wände ein freier, unbebauter Raum 10 (Figur 1) freigelassen, in welchen die Gasblasen aufsteigen und wo das Gemisch praktisch keinen Scherkräften mehr ausgesetzt ist. Noch besser ist dieses Bauprinzip in Figur 4 zu sehen, wo sich oberhalb der oberen Kanten der vertikalen Wände 5 ein völlig freier, grosser Raum 12 befindet. Vorzugsweise weist der Abschnitt 3 zum Abscheiden des Gasgemisches zwei Baueinheiten 7 und 8 auf. Dies deswegen, weil es vorteilhaft ist, das Abscheiden des Gasgemisches in einem ersten, in der ersten Baueinheit 7 vorgenommenen Schritt, und in einem zweiten in der zweiten Baueinheit 8 vorgenommenen Schritt durchzuführen.

Die erste Baueinheit 7 ist ein Rohrabschnitt 9. In diesem Rohr sind die Wände 5, die die engen Gänge bilden vertikal im Raum und parallel zueinander angeordnet, was besonders gut in der Figur 3 zu sehen ist. In dem Rohrabschnitt ist oben, oberhalb der Wände 5, die wie gezeigt, nicht bis zur oberen Abgrenzung im Rohr reichen, ein freier, unverbauter Raum 6 freigelassen. Wie oben bereits erwähnt, wird in diesem Raum das Gemisch, das hier durch die aufgestiegenen Gasblasen mit Gasen angereichert ist, nicht mehr einer intensiven Scherung unterzogen. In dieser Aufteilung wird das Gemisch in die zweite Baueinheit 8 übergeführt. Dabei wird darauf geachtet, dass z.B. durch eine starke Biegung des Verbindungsstückes zwischen diesen zwei Baueinheiten 7 und 8 es nicht zu einer nochmaligen Vermischung der Flüssigkeit mit dem Gas durch Einwirkung einer Zentrifugalkraft kommt. Deswegen soll diese Biegung so klein wie nur möglich sein und deswegen, wie es in der Figur 1 auch gezeigt ist, ist die erte Baueinheit 7 im Raum geneigt, um eben das Kleinhalten dieser Biegung zu ermöglichen.

Das Gemisch kommt nun in eine zweite Baueinheit 8, welche ein abgeschlossener Raum ist. In dem Raum ist ein konusförmiger Teil 11 glockenartig und vertikal im Raum angeordnet. An seiner Oberfläche sind auch die vertikalen Wände 5 aufgestellt. Sie sind entlang der gedachten Falllinien des konusförmigen Teiles 11 ausgerichtet und bilden so die erwähnten engen Gänge, die nach oben in einen leeren Raum 12 innerhalb der Baueinheit 8 offen sind. An diesen Raum 12 ist die Vorrichtung 4 angeschlossen zum Abführen des abgeschiedenen Gasgemisches. Da in dieser Konfiguration die vertikalen Wände nicht parallel zueinander verlaufen, ist es vorstellbar, und in machen Fällen vielleicht auch notwendig, an dem unteren Abschnitt der Gänge zusätzliche vertikal aufgestellte Zwischenwände vorzusehen, um die Gänge zu verengen und dadurch die entsprechende, intensive Scherung zwischen den Wänden 5 aufrecht zu erhal ten.

In der bereits erwähnten Versuchsanlage wurde in der zweiten Baueinheit 8 ebenfalls ein mittlerer Abstand von etwa 10 mm zwischen den Lamellen eingehalten.

Oben in der Baueinheit 8 ist oberhalb des konusförmigen Teiles 11 ein Rohrstück 13 vorgesehen, das so zwischen der ersten und zweiten Baueinheit 7 und 8 angeordnet ist. Der konusförmige Teil 11 reicht mit seiner Spitze in dieses Rohrstück 13 hinein. Dabei ist der konusförmige Teil 11 in vertikaler Richtung verschiebbar, so dass der Spalt, der zwischen dem unteren Rand 14 des Rohrstückes 13 und der Oberfläche des konusförmigen Teiles 11 gebildet ist, bezüglich seiner lichten Weite einstellbar ist. Diese Einstellung ist durch Verschiebung einer Stange 20 möglich, an welcher die Spitze des konusförmigen Teiles 11 befestigt ist. Diese Einstellung des Spaltes zwischen dem Rohrstück 13 und dem konusförmigen Teil 11 hat die Bedeutung, dass sich dadurch ein Druck- wie auch ein Geschwindigkeitsgefälle des durchfliessenden Fermentationsgemisches einstellen lässt. Um diese vertikale Verschiebung des konusförmigen Teiles 11 mit den Wänden 5 zu ermöglichen, sind in dem Rohrstück 13 vertikal verlaufende Schlitze 15, beginnend am unteren Rand 14 des Rohrstückes 13 ausgeführt. Die Teilung dieser Schlitze entspricht der Teilung der Wände 5 auf der Oberfläche des konusförmigen Teiles 11. Sie sind dimensioniert zum Durchgang der Wände 5 beim Einstellen des Spaltes zwischen dem Rohrstück 13 und der Oberfläche des konusförmigen Teiles 11 vorgesehen.

Es ist vorstellbar, dass die beschriebene Abscheidung der Gasphase aus dem Fermentationsgemisch wohl in gewissen Fällen schon durch die Wirkung der Scherkräfte an dem konusförmigen Teil 11 erreicht werden könnte. Es ist jedoch zu befürchten, dass insbesondere bei den hochviskosen, nicht-newton'schen Gemischen der Fluss den engen Gängen entlang eventuell entweder zu lange dauern würde, wobei eine kurze Verweilzeit gewünscht wird, oder dass es zu Abflussstörungen in den engen Gängen kommen könnte. Deswegen ist dieser Baueinheit 8 in diesem beschriebenen Fall die erste Baueinheit 7 vorgeschaltet, wo das Fermentationsgemisch einem gezwungenen Durchlauf unterzogen ist und wo dann eine sehr intensive Scherung und die erste Phase der Abscheidung der Gasphase passiert, bevor das Gemisch zur Beendigung des Abscheidens an dem konusförmigen Teil 11 anlangt.

Um den Fortgang in der zweiten Baueinheit 8 kontrollieren zu können, sind an dem Mantel der Baueinheit zwei Gucklöcher 21 vorgesehen.

Der Schutzbereich der vorliegenden Erfindung ist nicht durch die Beschreibung dieses ausgewählten Ausführungsbeispiels begrenzt. Es ist eine Reihe von Ausführungen vorstellbar, die auf dem hier beschriebenen und erklärten Prinzip beruhen, vorstellbar. So ist es auch denkbar, das Prinzip in Anordnungen, die auf dem Einsatz von Zentrifugalkräften beruhen, zum Tragen zu bringen. So ist z.B. vorstellbar, die erste Baueinheit 7 mit den vertikal aufgestellten Wänden, wie es in Figur 3 dargestellt ist, zu einer Zickzack-Passage umzuformen, wodurch dann in den Wendungen des Zickzack-Weges zur intensiveren Scherung des Fermentationsstoffes durch Einwirkungen der auftretenden Zentrifugalkräfte bei einer beachtlichen Durchflussgeschwindigkeit des Fermentationsstoffes käme.

**Patentansprüche**

1. Fermentationsanlage, aufweisend eine Rohrschlaufe (1) für einen durch eine Umwälzpumpe (16) erzwungenen Kreislauf eines flüssigen, hochviskosen, nicht-newton'schen (pseudoplastischen) Fermentationsgemisches, welche einen Abschnitt (2) zum Begasen und Mischen des Fermentationsgemisches, einen Abschnitt (3) zum Abscheiden eines bei dem Begasen und Fermentieren entstehenden Gasgemisches aus dem Fermentationsgemisch und eine Vorrichtung (4) zum Abführen des Gasgemisches aus der Fermentationsanlage aufweist, dadurch gekennzeichnet, dass in dem Abschnitt (3) zum Abscheiden des Gasgemisches Einbauten (5) vorgesehen sind zum Scheren des Gas/Flüssig-Gemisches in engen, vertikalen, nach oben offenen Gängen zum Herabsetzen der Viskosität des Gemisches, so dass die im Gemisch dispergierten Gase durch die Gemischschicht mit eigenem Auftrieb im Raum nach oben steigen, in einen Sammelraum (12), aus welchem sie durch die Vorrichtung (4) zum Abführen des Gasgemisches aus dem Kreislauf weggeleitet werden.

2. Fermentationsanlage nach Anspruch 1, dadurch gekennzeichnet, dass die Einbauten vertikal im Raum angeordnete Wände (5) aufweisen, zwischen denen die engen, nach oben offenen Gänge gebildet sind.

3. Fermentationsanlage nach Anspruch 1, dadurch gekennzeichnet, dass der Abschnitt (3) zum Abscheiden des Gasgemisches zwei Baueinheiten (7 und 8) aufweist, zum Abscheiden des Gasgemisches in einem ersten (7) und in einem zweiten (8) Schritt.

4. Fermentationsanlage nach Anspruch 3, dadurch gekennzeichnet, dass die erste Baueinheit (7) ein Rohrabschnitt ist, in welchem die Gänge bildenden Wände (5) vertikal im Raum und parallel zueinander angeordnet sind, wobei in dem Rohrabschnitt (7) oben, oberhalb der Wände (5) ein freier, unverbauter Raum (6) freigelassen ist.

5. Fermentationsanlage nach Anspruch 3, dadurch gekennzeichnet, dass die zweite Baueinheit (8) einabgeschlossener Raum ist, in dem ein konusförmiger Teil (11) glockenartig vertikal im Raum angeordnet ist und an seiner Oberfläche die vertikalen Wände (5) aufgestellt hat, die entlang der gedachten Falllinien des konusförmigen Teiles (11) verlaufen, die enge Gänge bilden und nach oben in einen leeren Raum (12) offen sind, an welchen die Vorrichtung (4) zum Abführen des Gasgemisches angeschlossen ist.

6. Fermentationsanlage nach Anspruch 5, dadurch gekennzeichnet, dass oberhalb des konusförmigen Teiles (11) ein Rohrstück (13) vorgesehen ist, in welches die Spitze des konusförmigen Teiles (11) hineinreicht, und wobei der konusförmige Teil (11) in vertikaler Richtung verschiebbar ist, so dass der Spalt, der zwischen dem unteren Rand (14) des Rohrstückes (15) und der Oberfläche des konusförmigen Teiles (11) gebildet ist, bezüglich seiner lichten Weite einstellbar ist.

7. Fermentationsanlage nach Anspruch 6, dadurch gekennzeichnet, dass in dem Rohrstück (13) vertikale, am unteren Rand (14) des Rohrstückes (13) anfangende Schlitze (15) ausgeführt sind, deren Teilung der Teilung der vertikal stehenden Wände (5) auf dem konusförmigen Teil (11) entspricht, und die zum Durchgang der Wände (5) beim vertikalen Verschieben des konusförmigen Teiles (11) beim Einstellen des Spaltes zwischen dem unteren Rand (14) des Rohrstückes (13) und der Oberfläche des konusförmigen Teiles (11) vorgesehen sind.

**Revendications**

1. Installation de fermentation pourvue d'une boucle tubulaire (1) pour la circulation, forcée sous l'action d'une pompe (16), d'un mélange liquide fermentescible très visqueux, non newtonien (pseudoplastique), qui présente une section (2) d'aération et de brassage du mélange fermentescible, une section (3) permettant de séparer du mélange fermentescible un mélange gazeux produit lors de l'aération et de la fermentation, et un dispositif (4) pour décharger de l'installation de fermentation le mélange gazeux, caractérisée par le fait que des chicanes (5) sont prévues dans la section (3) de séparation du mélange gazeux pour cisailler le mélange gaz/liquide dans des couloirs verticaux étroits ouverts vers le haut afin de réduire la viscosité du mélange, de manière que les gaz dispersés dans le mélange s'élèvent à travers la couche de mélange par leur propre force ascensionnelle pour se rassembler dans un espace (12) duquel ils sont déchargés du circuit par le dispositif (4) d'évacuation du mélange gazeux.

2. Installation de fermentation suivant la revendication 1, caractérisée en ce que les chicanes présentent des parois (5) disposées verticalement dans l'espace, entre lesquelles les couloirs étroits ouverts vers le haut sont formés.

3. Installation de fermentation suivant la revendication 1, caractérisée en ce que la section (3) servant à la séparation du mélange gazeux présente deux unités de construction (7) et (8) destinées à séparer le mélange gazeux dans une première section (7) et une seconde section (8).

4. Installation de fermentation suivant la revendication 3, caractérisée en ce que le premier élément de construction (7) est une section de tuyau dans laquelle les parois (5) formant des couloirs sont disposées verticalement dans l'espace et parallèlement les unes aux autres, un espace libre (6) sans élément de construction étant ménagé dans la section de tuyau (7) à la partie supérieure, au-dessus des parois (5).

5. Installation de fermentation suivant la revendication 3, caractérisée en ce que la seconde unité de construction (8) est un espace clos dans lequel une partie conique (11) est disposée verticalement à la manière d'une cloche et porte, disposées à sa surface, les parois verticales (5) qui s'étendent dans la direction des lignes de pente imaginaires de la partie conique (11), forment les couloirs étroits et s'ouvrent vers le haut dans un espace libre (12) auquel se raccorde le dispositif (4) servant à la décharge du mélange gazeux.

6. Installation de fermentation suivant la revendication 5, caractérisée en ce qu'il est prévu audessus de la partie conique (11) une pièce tubulaire (13) dans laquelle s'engage la pointe de la partie conique (11), et cette dernière peut être déplacée dans la direction verticale, de manière que l'intervalle qui est formé entre le bord inférieur (14) de la pièce tubulaire (15) et la surface de la partie conique (11) puisse être réglé quant à sa largeur intérieure.

7. Installation de fermentation suivant la revendication 6, caractérisée par la réalisation, dans la pièce tubulaire (13), de fentes verticales (15) débutant au bord inférieur (14) de la pièce tubulaire (13), dont la division correspond à celle des parois verticales (5) sur la partie conique (11), et qui sont prévues pour le passage des parois (5) lors du déplacement vertical de la partie conique (11) au moment du réglage de l'intervalle entre le bord inférieur (14) de la pièce tubulaire (13) et la surface de la partie conique (11).

**Claims**

1. Fermentation plant, comprising a looped pipe (1) for the circulation, forced by a circulating pump (16), of a fluid, highly viscous, non-Newtonian (pseudoplastic) fermentation mixture, which pipe comprises a section (2) for gassing and mixing the fermentation mixture, a section (3) for separating a gas mixture resulting from the gassing and fermentation from the fermentation mixture and a device (4) for removing the gas mixture from the fermentation plant, characterised in that fitted components (5) are provided in the section (3) for separating the gas mixture in order to shear the gas/liquid mixture in narrow, vertical passages, which are open at the top, to reduce the viscosity of the mixture, so that the gases which are dispersed in the mixture rise through the mixture layer due to their own buoyancy into a collecting space (12), out of which they are conveyed by the device (4) for removing the gas mixture from the circulation.

2. Fermentation plant according to claim 1, characterised in that the fitted components comprise vertical walls (5), between which the narrow passages, which are open at the top, are formed.

3. Fermentation plant according to claim 1, characterised in that the section (3) for separating the gas mixture comprises two structural units (7) and (8) for separating the gas mixture in a first (7) and in a second (8) step.

4. Fermentation plant according to claim 3, characterised in that the first structural unit (7) is a pipe section in which the walls (5) forming the passages are arranged vertically and parallel to one another, a free, unobstructed space (6) being left at the top of the pipe section (7) above the walls (5).

5. Fermentation plant according to claim 3, characterised in that the second structural unit (8) is an enclosed space in which a conical part (11) is arranged vertically and in the manner of a bell, with the vertical walls (5) mounted on its surface, which walls extend along the imaginary lines of slope of the conical part (11), form the narrow passages and open at the top into an empty space (12), with which the device (4) communicates to remove the gas mixture.

6. Fermentation plant according to claim 5, characterised in that a pipe length (13) is provided above the conical part (11), into which length the peak of the conical part (11) extends, the latter being displaceable in the vertical direction, so that the clear width of the gap which is formed between the lower edge (14) of the pipe length (13) and the surface of the conical part (11) can be adjusted.

7. Fermentation plant according to claim 6, characterised in that vertical slots (15), which commence at the lower edge (14) of the pipe length (13), are provided in the latter, the spacing of which slots corresponds to the spacing of the vertical walls (5) on the conical part (11) and which are provided to permit the walls (5) to pass through during the vertical displacement of the conical part (11) when the gap between the lower edge (14) of the pipe length (13) and the surface of the conical part (11) is adjusted.

Fig. 2

Fig. 3

Fig.1

Fig. 4